# EUROPEAN PATENT SPECIFICATION

(11) **EP 0 714 317 B1**
(45) Date of publication and mention of the grant of the patent: **04.09.2002**
(21) Application number: 95923755.3
(22) Date of filing: 07.06.1995
(51) Int. Cl.: A61N 1/36, A61N 1/375

(54) **IMPLANTABLE CERAMIC DEVICE ENCLOSURE**
IMPLANTIERBARES KERAMISCHES GERÄTEGEHÄUSE
BOITIER EN CERAMIQUE POUR DISPOSITIF IMPLANTABLE

(30) Priority: 16.06.1994 US 260639; 16.06.1994 US 260866
(43) Date of publication of application: 05.06.1996
(73) Proprietor: MEDTRONIC, INC., Minneapolis, Minnesota 55432-3576 (US)
(72) Inventor: HASSLER, Beth Anne, White Bear Lake, MN 55110 (US); DONDERS, Adrianus P., Andover, MN 55304 (US)
(74) Representative: Hughes, Andrea Michelle
(86) International application number: US9507262
(87) International publication number: WO95034342

(56) References cited:
- DE-A- 2 750 164
- US-A- 4 614 194
- US-A- 4 616 655
- US-A- 4 785 827
- US-A- 4 991 582
- J.A. SARTELL "Electronic Packaging: Materials and Processes, Proceedings of ASM's 2nd Electronic Packaging: Materials and Processes Conference", published 1985 by ASM, "Fast Turnaround Multilayer Cofired Ceramic Motherboard Fabrications" pages 117-121, page 117; fig. 1 (cited in the application).

## Description

The present invention relates to the packaging of implantable medical devices such as artificial cardiac pacemakers and the like.

Generally speaking, a cardiac pacemaker or implantable pulse generator (IPG) is an electrical device used to supplant some or all of an abnormal heart's natural pacing function, by delivering appropriately timed electrical stimulation signals designed to cause the myocardium of the heart to contract or "beat".

Using telemetry, modern pacemakers are often programmable with regard to data and functionality prior to, and even after implant. Typical pacemakers are enclosed by metal casings such as titanium, which has good body compatibility. However, metal enclosures often cause interference during telemetry.

To create pacemakers and other implantable medical devices with enclosures which are transparent to radio frequency (RF) waves during telemetry, the enclosure can be constructed of ceramic material, for example. Such is the approach of U.S. Patent No. 4,785,827 issued to Fischer, and U.S. Patent No. 4,991,582 issued to Byers et al.

Implantable medical devices of the above-mentioned type have a hybrid circuit with feedthroughs attached thereto and leading through a glass-to-metal feedthrough substrate to the connector block for electrical coupling to a lead (for stimulating, sensing, or both functions). As a result of the construction of prior art substrates, fewer feedthroughs can be handled than is desirable, and the cost of producing such substrates is expensive.

In view of the foregoing, it is a first object of the present invention to provide an implantable medical device which is nearly transparent to radio frequency waves for telemetering purposes, especially in the 400 kilohertz to 40 megahertz frequency range.

It is a second object of the present invention to provide an implantable medical device wherein its feedthrough substrate provides for a higher density of feedthroughs.

It is a third object of the present invention to provide an implantable medical device wherein its feedthrough substrate is less expensive than prior art feedthrough substrates.

The present invention according to a first aspect provides a packaging arrangement for the outer packaging of an implantable medical device comprising:
a ceramic enclosure having an opening to receive circuitry of said implantable medical device; and
   characterised by including
a multi-layered feedthrough substrate adapted to be coupled to said ceramic enclosure at edges around said opening, said substrate having multiple feedthroughs for electrically coupling said circuitry inside said enclosure to the outside of said enclosure.

The present invention according to a second aspect provides a packaging arrangement for the outer packaging of an implantable medical device comprising:
at least a first enclosure shell; and
at least a second enclosure shell;
wherein said enclosure shells are joinable to sealably enclose components of said implantable medical device; and characterised in that said first and second enclosure shells are multi-layered, and layers of said enclosure shells are adapted to conduct signals between implantable medical device components mounted directly on at least one of said shells.

The present invention thus provides an implantable medical device satisfying the above objects wherein its enclosure is ceramic.

Further, the present invention provides an implantable medical device wherein the enclosure walls are electrically conducting.

The details of the present invention will be revealed in the following description of preferred embodiments, given by way of example only, with reference to the drawings.

The various figures of the drawings are briefly described as follows:
Figure 1 is an exploded isometric view of a prior art pacemaker with a ceramic enclosure;
Figure 2 is an exploded isometric view of a pacemaker employing the present-inventive features; and
Figure 3 is an exploded isometric view of the pacemaker in Figure 2, additionally showing a connector block and a pacemaker lead.
Figure 4 is a isometric view of a pacemaker employing the present-inventive ceramic enclosure;
Figure 5 is an isometric view of the battery-carrying half of a ceramic enclosure according to the present invention.
Figure 6 is an isometric view of the hybrid circuit and feedthrough-carrying half of a ceramic enclosure according to the present invention; and
Figure 7 is an exploded isometric view of the pacemaker in Figure 4, without a connector block.

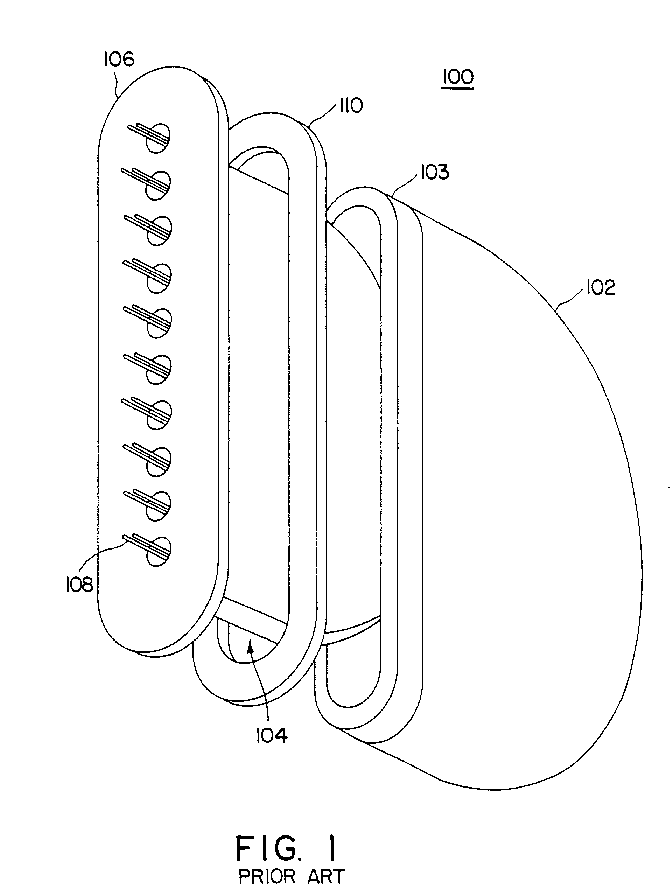

Figure 1 shows a prior art packaging arrangement/scheme 100 for a pacemaker. The arrangement 100 has a ceramic enclosure 102 with a metalized portion 103. A hybrid circuit 104 is attached to a feedthrough substrate 106 which is a glass-to-metal feedthrough assembly. Feedthroughs 108 (metal) electrically connect to components of the hybrid circuit 104 at one end, and are adapted to electrically connect a connector block (not shown) at the other (exposed) end. A weld ring 110 is welded on one side to the enclosure 102 (at the metalized portion 103), and on its other side to the glass-to-metal feedthrough 106. The entire packaging provides an implantable medical device which is hermetically sealed.

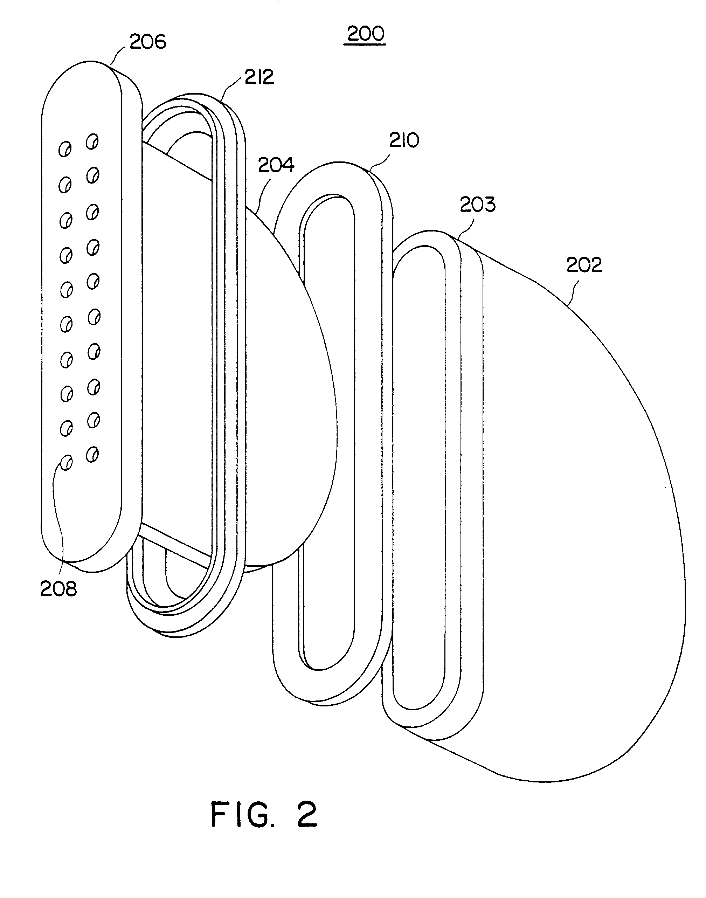

The present-inventive implantable medical device packaging arrangement/scheme 200 is illustrated in Figure 2. The ceramic enclosure 202 is composed of biocompatible 99.5 percent aluminum oxide in the preferred embodiment, which has been shown to have good tissue compatibility. A metalized portion 203 is formed by sputtering (as is known in the art) a thin film of niobium on the ceramic surface.

A hybrid circuit 204 is connected to a novel multi-layered feedthrough substrate 206. The substrate 206 is composed of several layers of ceramic material with metal-plated input/output vias used to electrically connect the various layers. The plating metals may be gold and nickel, for example. As a result of the multi-layer and via configuration, a higher density of feedthroughs 208 is possible over the glass-to-metal feedthrough substrate (element 106 in Figure 1) approaches in the prior art. The substrate 206 can be constructed using techniques known in the art, such as is disclosed by Beth A. Hassler in "Fast Turnaround Multilayer Cofired Ceramic Motherboard Fabrication," *Proceedings of ASM's 2nd Electronic Packaging: Materials and Processes Conference* (October 1985): 117-121.

Two weld rings 210 and 212 are chosen to have thermal expansion characteristics sufficiently similar to the ceramic material used to maintain good bonding over a broad temperature range. Weld ring 210 is brazed to the metalized portion 203 of the ceramic enclosure 202, and welded to the weld ring 212. Weld ring 212 is also brazed to the multi-layered feedthrough 206. The welded and brazed surfaces complete a hermetic seal of the pacemaker.

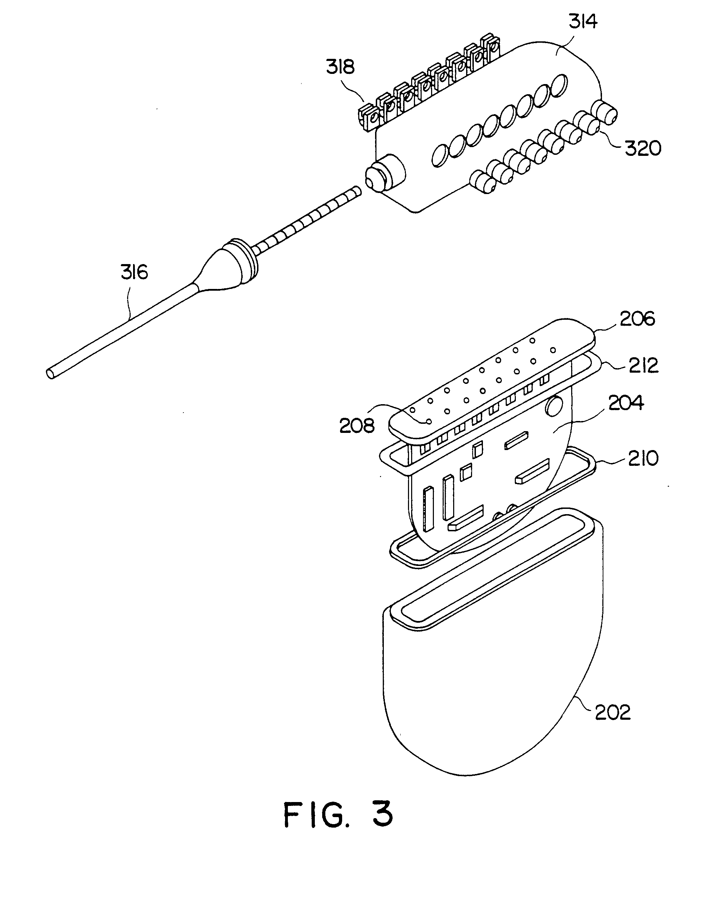

Figure 3 shows the pacemaker in Figure 2 with additional components. Namely, the pacemaker also has a connector block 314 which is fastened to the multi-layered feedthrough substrate 206. The connector block 314 electrically connects one or more pacemaker leads 316 to the feedthroughs 208 via lead clamps 318. The lead 316 is firmly held in place (in the lead clamps 318) by tightening set screws 320.

Thus, electrical connection is made from the lead 316 to the connector block 314 to the feedthroughs 208 to the hybrid circuitry 204.

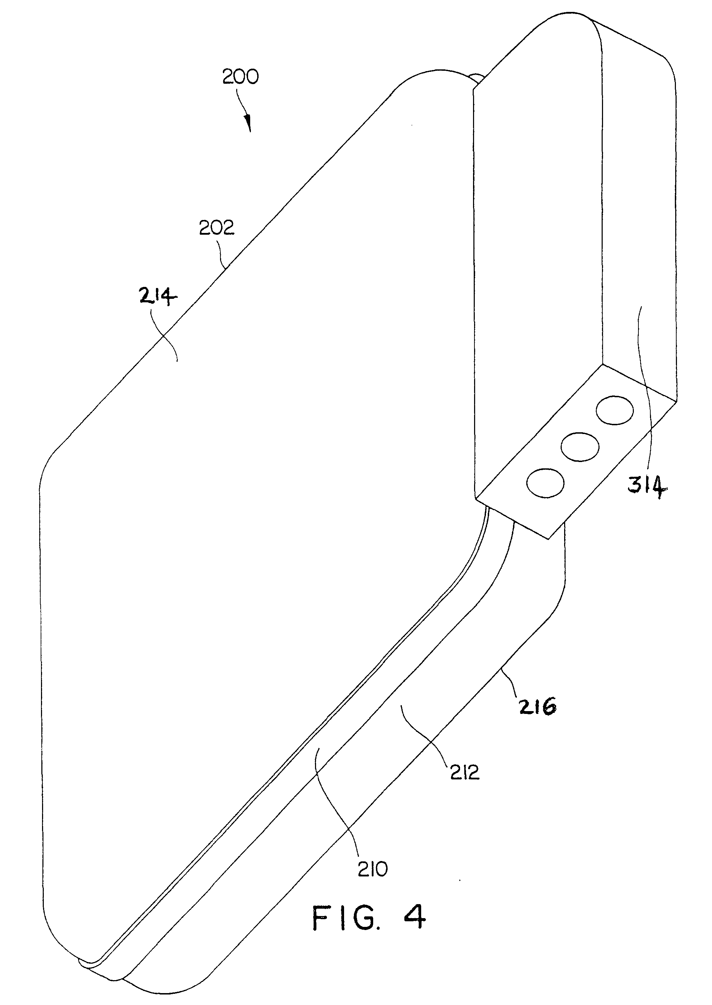

Figure 4 shows an implantable medical device 200 employing the ceramic enclosure 202 of the present invention. In this case a pacemaker has two ceramic enclosure halves (or shells) 214 and 216 which are joined by weld rings 210 and 212 to form a hermetic seal. Each half 214 and 216 has a thin metalized layer for brazing the weld rings 210 and 212 thereto, respectively.

A connector block 314 is attached to the enclosure 202. In the preferred embodiment, the enclosure half 214 carries a battery (not shown) mounted to its inside wall, while the enclosure half 216 carries both a hybrid electronic circuit and feedthroughs (see Figure 4) for connecting to the connector block 314. The feedthroughs provide an electrical connection between the hybrid circuitry and stimulation and sensing leads when attached to the connector block 314.

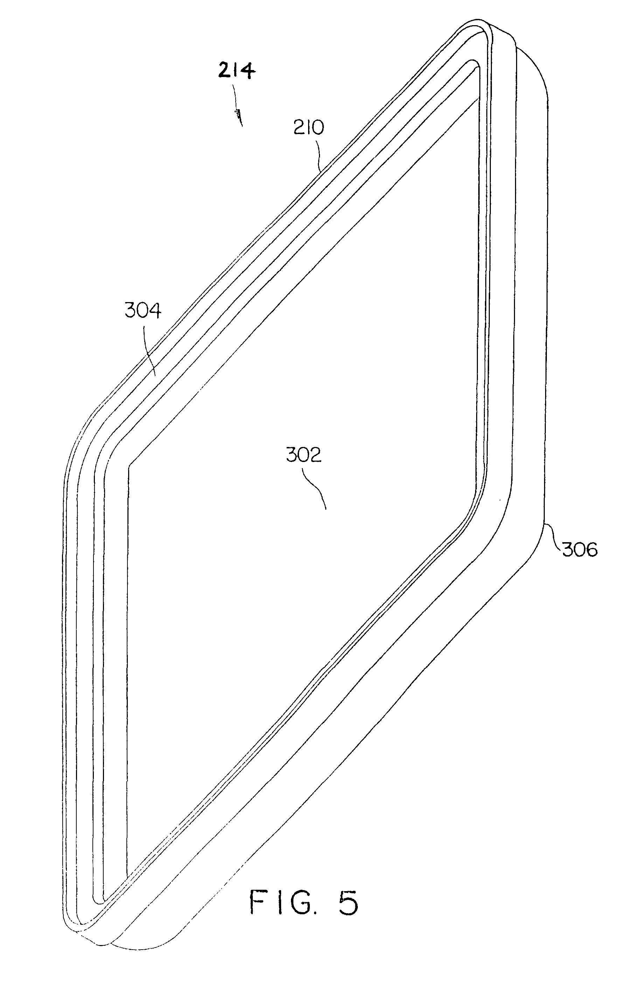

The enclosure half 214 is shown in greater detail in Figure 5. The walls of the ceramic enclosure halves 214 and 216 are constructed of multiple ceramic layers (302 in Figure 3), variously containing electrical conduction areas, vias for inter-layer communication, and electromagnetic interference (EMI) shielding areas. The layers may be constructed of biocompatible 99.5 percent aluminum oxide, for example, which has been shown to have good tissue compatibility.

The ceramic enclosure shells 214 and 216 can be constructed using techniques known in the art, such as is disclosed in the previously mentioned article by Beth A. Hassler, in "Fast Turnaround Multilayer Cofired Ceramic Motherboard Fabrication".

Thin ceramic layers are joined, and then form-molded at the corners (e.g., 306). A metalized portion 304 is formed by sputtering (as is known in the art) a thin film of niobium on the ceramic surface. This provides good braze-bonding characteristics for attaching the ceramic wall to the weld ring 210. The weld ring 212 is attached to the enclosure half 216 in the same manner.

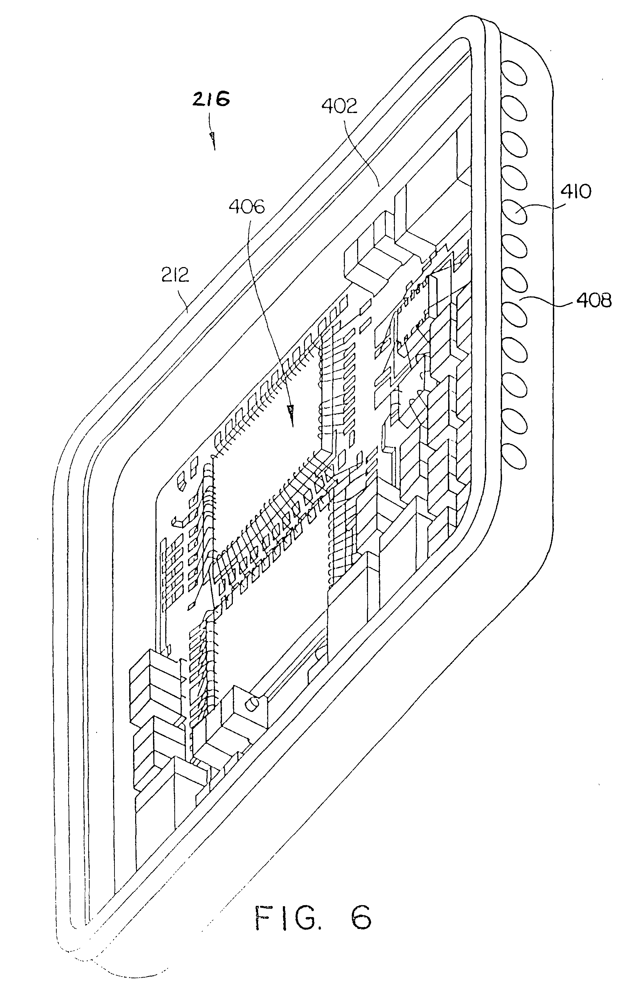

The enclosure half 216 is detailed in Figure 6. The multi-layer nature of the wall 402 of the ceramic enclosure half 216 allows the hybrid circuitry 406 of the pacemaker to be mounted directly on the ceramic enclosure, thereby saving time, money and parts compared with prior art ceramic enclosures (which do not electrically connect the enclosed components).

In addition to the hybrid circuitry, the enclosure half 216 also has a feedthrough area 408. The several layers of the ceramic material have metal-plated input/output vias used to electrically connect the various layers. The plating metals may be gold and nickel, for example. As a result of the multi-layer and via configuration, a higher density of feedthroughs 410 are possible over the glass-to-metal substrate (element 106 Figure 1) approaches in the prior art. The feedthroughs 410 electrically connect the circuitry carried by the enclosure to the connector block 314.

The weld ring 210 is chosen to have thermal expansion characteristics sufficiently similar to the ceramic material used to maintain good bonding over a broad temperature range.

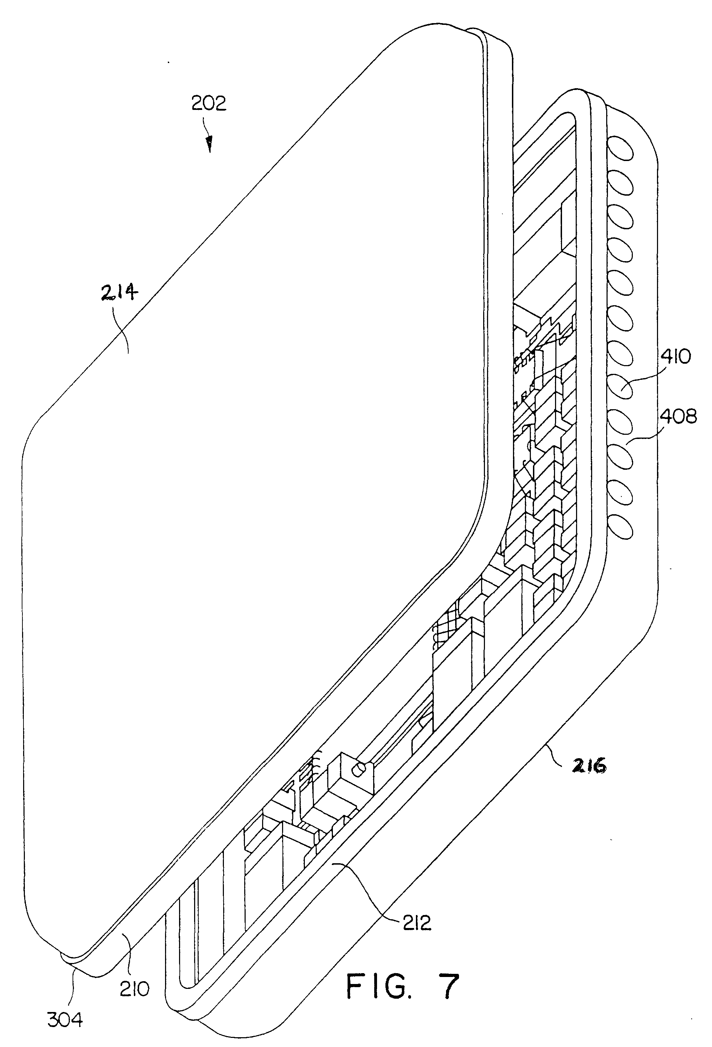

Figure 7 shows an exploded isometric view of the ceramic enclosure 202 for illustrative purposes.

Variations and modifications to the present invention are possible given the above disclosure. However, such variations and modifications are intended to be within the scope of the invention claimed by this letters patent. For example, the packaging arrangement described *supra*. is optimal for bipolar pacing. The ceramic enclosure 202 may be coated with a thin metal layer (using sputtering techniques, for example) to enable unipolar pacing. Also bonding of the enclosure shells of the present invention need not be limited to the use of brazing and welding techniques.

## Claims

1. A packaging arrangement for the outer packaging of an implantable medical device comprising:
a ceramic enclosure (202) having an opening to receive circuitry (204) of said implantable medical device; and **characterised by** including
a multi-layered feedthrough substrate (206) adapted to be coupled to said ceramic enclosure (202) at edges around said opening, said substrate (206) having multiple feedthroughs (208) for electrically coupling said circuitry (204) inside said enclosure to the outside of said enclosure.

2. The packaging arrangement of claim 1 further comprising a connector block (314) for coupling to said substrate (206), and for coupling to at least one electrical lead (316), said connector block electrically connecting said lead (316) to said feedthroughs (208).

3. The packaging arrangement in claim 1 or 2, further comprising:
a first weld ring (210) for coupling to said ceramic enclosure (202);
a second weld ring (212) for coupling to said first weld ring (210), and to said multi-layered feedthrough substrate (206).

4. The packaging arrangement in claims 1, 2 or 3 wherein said ceramic enclosure (202) further comprises a metallic outer layer (203).

5. A packaging arrangement for the outer packaging of an implantable medical device comprising:
at least a first enclosure shell (214); and
at least a second enclosure shell (216) ;
wherein said enclosure shells (214, 216) are joinable to sealably enclose components (406) of said implantable medical device; and **characterised in that** said first and second enclosure shells (214, 216) are multi-layered, and layers of said enclosure shells are adapted to conduct signals between implantable medical device components (406) mounted directly on at least one of said shells (216).

6. The packaging arrangement of claim 5 wherein the layers of said enclosure shells (214, 216) are ceramic.

7. The packaging arrangement of claim 5 or 6 wherein at least one of said enclosure shells comprises a multi-layered feedthrough (408) having multiple feedthroughs (410) for electrically coupling circuitry (406) mounted on said shell to the outside of said shell.

8. The packaging arrangement of claim 5, 6 or 7 wherein said first enclosure shell is adapted to carry a hybrid circuit (406) of a pacemaker, and said second enclosure shell is adapted to carry a battery circuit of said pacemaker.

9. The packaging arrangement of any of claims 5 to 8 further comprising a connector block for coupling to said enclosure shells.

10. The packaging arrangement of any of claims 5 to 9 wherein said enclosure shells further comprise an outer metallic layer.

## Patentansprüche

1. Einkapselungsanordnung bzw. Verpackungsanordnung für die äußere Einkapselung einer implantierbaren medizinischen Vorrichtung, die
eine keramische Umschließung (202) umfaßt, die eine Öffnung besitzt, um eine Schaltungsanordnung (204) der implantierbaren medizinischen Vorrichtung aufzunehmen; und **gekennzeichnet ist durch**
ein Mehrlagen-Durchführungssubstrat (206), das mit der keramischen Umschließung (202) an um die Öffnung verlaufenden Kanten gekoppelt werden kann, wobei das Substrat (206) mehrere Durchführungen (208) besitzt, um die Schaltungsanordnung (204) in der Umschließung mit der äußeren Umgebung der Umschließung elektrisch zu koppeln.

2. Einkapselungsanordnung nach Anspruch 1, die ferner einen Verbinderblock (314) umfaßt, der mit dem Substrat (206) und mit wenigstens einer elektrischen Leitung (316) gekoppelt ist, wobei der Verbinderblock die Leitung (316) mit den Durchführungen (208) elektrisch verbindet.

3. Einkapselungsanordnung nach Anspruch 1 oder 2, die ferner umfaßt:
einem ersten Schweißring (210) für die Kopplung mit der keramischen Umschließung (202);
einem zweiten Schweißring (212) für die Kopplung mit dem ersten Schweißring (210) und mit dem Mehrlagen-Durchführungssubstrat (206).

4. Einkapselungsanordnung nach den Ansprüchen 1, 2 oder 3, bei der die keramische Umschließung (202) ferner eine metallische Außenschicht (203) umfaßt.

5. Einkapselungsanordnung für die äußere Einkapselung einer implantierbaren medizinischen Vorrichtung, die umfaßt:
ein erstes Umschließungsgehäuse (214); und
wenigstens ein zweites Umschließungsgehäuse (216);
wobei die Umschließungsgehäuse (214, 216) verbunden werden können, um Komponenten (406) der implantierbaren medizinischen Vorrichtung dicht zu umschließen; und **dadurch gekennzeichnet, daß** die ersten und zweiten Umschließungsgehäuse (214, 216) mehrlagig sind und Lagen der Umschließungsgehäuse so beschaffen sind, daß sie Signale zwischen direkt auf wenigstens einem der Gehäuse (216) montierten Komponenten (406) der implantierbaren medizinischen Vorrichtung leiten können.

6. Einkapselungsanordnung nach Anspruch 5, bei der die Lagen der Umschließungsgehäuse (214, 216) aus Keramik sind.

7. Einkapselungsanordnung nach Anspruch 5 oder 6, bei der wenigstens eines der Umschließungsgehäuse eine Mehrlagen-Durchführung (408) mit mehreren Durchführungen (410) für die elektrische Verbindung der am Gehäuse montierten Schaltungsanordnung (406) mit der äußeren Umgebung des Gehäuses umfaßt.

8. Einkapselungsanordnung nach den Ansprüchen 5, 6 oder 7, bei der das erste Umschließungsgehäuse so beschaffen ist, daß es eine Hybridschaltung (406) eines Herzschrittmachers trägt, und das zweite Umschließungsgehäuse so beschaffen ist, daß es eine Batterieschaltung des Herzschrittmachers trägt.

9. Einkapselungsanordnung nach einem der Ansprüche 5 bis 8, die ferner einen Verbinderblock umfaßt, der mit den Umschließungsgehäusen gekoppelt ist.

10. Einkapselungsanordnung nach einem der Ansprüche 5 bis 9, bei der die Umschließungsgehäuse ferner eine äußere Metallschicht aufweisen.

## Revendications

1. Dispositif de conditionnement pour le conditionnement extérieur d'un dispositif médical implantable comprenant :
un boîtier en céramique (202) ayant une ouverture pour recevoir des circuits (204) dudit dispositif médical implantable; et **caractérisé en ce qu'**il comprend :
un substrat de traversée à couches multiples (206) adapté pour être couplé audit boîtier en céramique (202) sur les bords autour de ladite ouverture, ledit substrat (206) ayant de multiples traversées (208) pour coupler électriquement lesdits circuits (204) à l'intérieur dudit boîtier à l'extérieur dudit boîtier.

2. Dispositif de conditionnement selon la revendication 1, comprenant en outre un bloc connecteur (314) pour un couplage avec ledit substrat (206) et pour un couplage avec au moins un conducteur électrique (316), ledit bloc connecteur connectant électriquement ledit conducteur (316) auxdites traversées (208).

3. Dispositif de conditionnement selon la revendication 1 ou 2, comprenant en outre :
un premier anneau de soudage (210) pour un couplage avec ledit boîtier en céramique (202),
un second anneau de soudage (212) pour un couplage avec ledit premier anneau de soudage (210) et avec ledit substrat de traversée à couches multiples (206).

4. Dispositif de conditionnement selon les revendications 1, 2 ou 3, dans lequel ledit boîtier en céramique (202) comprend également une couche métallique externe (203).

5. Dispositif de conditionnement pour le conditionnement extérieur d'un dispositif médical implantable comprenant :
au moins une première gaine de boîtier (214); et
au moins une seconde gaine de boîtier (216);
dans lequel lesdites gaines de boîtier (214, 216) peuvent être jointes pour enserrer hermétiquement des composants (406) dudit dispositif médical implantable; et **caractérisé en ce que** lesdites première et seconde gaines de boîtier (214, 216) sont à couches multiples, et les couches desdites gaines de boîtier sont adaptées pour acheminer des signaux entre les composants du dispositif médical implantable (406) montés directement sur au moins l'une desdites gaines (216).

6. Dispositif de conditionnement selon la revendication 5, dans lequel les couches desdites gaines de boîtier (214, 216) sont en céramique.

7. Dispositif de conditionnement selon la revendication 5 ou 6, dans lequel au moins l'une desdites gaines de boîtier comprend une traversée à couches multiples (408) ayant de multiples traversées (410) pour coupler électriquement les circuits (406) montés sur ladite gaine à l'extérieur de ladite gaine.

8. Dispositif de conditionnement selon la revendication 5, 6 ou 7, dans lequel ladite première gaine de boîtier est adaptée pour porter un circuit hybride (406) d'un stimulateur cardiaque et ladite seconde gaine de boîtier est adaptée pour porter un circuit de piles dudit stimulateur cardiaque.

9. Dispositif de conditionnement selon l'une quelconque des revendications 5 à 8, comprenant en outre un bloc connecteur pour un couplage avec lesdites gaines de boîtier.

10. Dispositif de conditionnement selon l'une quelconque des revendications 5 à 9, dans lequel lesdites gaines de boîtier comprennent en outre une couche métallique externe.
